# EUROPEAN PATENT SPECIFICATION

(11) **EP 3 409 454 B1**
(45) Date of publication and mention of the grant of the patent: **01.03.2023**
(21) Application number: 17743970.0
(22) Date of filing: 13.01.2017
(51) Int. Cl.: B29C 64/112, B29C 64/124, B33Y 80/00, B33Y 70/00, B33Y 10/00, A61L 27/44, A61L 27/52

(54) **THREE-DIMENSIONAL MOLDED OBJECT, METHOD FOR PRODUCING THREE-DIMENSIONAL MOLDED OBJECT, MATERIAL SET FOR THREE-DIMENSIONAL MOLDING, AND HYDROGEL PRECURSOR LIQUID**
DREIDIMENSIONALES FORMOBJEKT, VERFAHREN ZUR HERSTELLUNG EINES DREIDIMENSIONALEN FORMOBJEKTS, MATERIALSATZ ZUM DREIDIMENSIONALEN FORMEN UND HYDROGELVORLÄUFERFLÜSSIGKEIT
OBJET MOULÉ TRIDIMENSIONNEL, PROCÉDÉ DE PRODUCTION D'UN OBJET MOULÉ TRIDIMENSIONNEL, ENSEMBLE DE MATÉRIAUX POUR MOULAGE TRIDIMENSIONNEL ET LIQUIDE PRÉCURSEUR D'HYDROGEL

(30) Priority: 25.01.2016 JP 2016011897
(43) Date of publication of application: 05.12.2018
(73) Proprietor: Ricoh Company, Ltd., Tokyo 143-8555 (JP)
(72) Inventor: MATSUMURA, Takashi, Tokyo 143-8555 (JP); NORIKANE, Yoshihiro, Tokyo 143-8555 (JP); NIIMI, Tatsuya, Tokyo 143-8555 (JP); IWATA, Hiroshi, Tokyo 143-8555 (JP)
(74) Representative: Marks & Clerk LLP
(86) International application number: PCT/JP2017/001028
(87) International publication number: WO 2017/130739

(56) References cited:
- EP-A1- 2 793 969
- EP-B1- 2 793 969
- WO-A1-2014/197999
- JP-A- H0 732 493
- JP-A- 2015 136 895
- JP-A- 2016 010 870
- US-A1- 2008 226 724

## Description

### Technical Field

The present invention relates to a three-dimensional object, a three-dimensional object producing method, and a hydrogel precursor liquid.

### Background Art

Hitherto, as a laminated object manufacturing method, there has been proposed a method of irradiating a photo-curable resin in a liquid state with laser light, particularly, an ultraviolet ray of light to form one layer of the resin after another to produce a three-dimensional stereoscopic object (for example, see Patent document 1).

Further, in recent years, there has been disclosed an inkjet stereolithography method of forming an image of a needed portion of an object with a photo-curable resin in a liquid state by an inkjet method, and laminating such images to form a three-dimensional object. For such an inkjet stereolithography method, there have been proposed methods of simultaneously forming a support different from the object to prevent deformation or fall of the three-dimensional object during object production (for example, see Patent documents 2 and 3).

Moreover, there have been increasing needs for gel-state or soft three-dimensional objects having three-dimensional, accurate structures such as medical organ models and cell scaffolding materials used in the regenerative medicine. There has been proposed a method for producing such gel-state or soft three-dimensional objects (for example, see Patent document 4).

Reference is also made to EP 2793969.

### Citation List

### Patent Document

Patent document 1: Japanese Translation of PCT International Application Publication No. JP-T-2009-519143
Patent document 2: Japanese Patent No. 4366538
Patent document 3: Japanese patent No. 4908679
Patent document 4: Japanese Unexamined Patent Application Publication No. 2015-136895

### Summary of Invention

### Technical Problem

The present invention has an object to provide a complicated, accurate three-dimensional object having an excellent compressive strength.

### Solution to Problem

As a means of realizing the object described above, a three-dimensional object is formed of a hydrogel containing water as a main component. The three-dimensional object contains a chelate agent.

### Effects of the Invention

The present invention can provide a complicated, accurate three-dimensional object having an excellent compressive strength.

### Brief Description of the Drawings

FIG. 1 is a schematic view illustrating an example of a three-dimensional object producing apparatus of the present invention;
FIG. 2 is a schematic view illustrating another example of a three-dimensional object producing apparatus of the present invention;
FIG. 3 is a schematic view illustrating another example of a three-dimensional object producing apparatus of the present invention;
FIG. 4 is a view illustrating a state of an intermediate body (before detachment of a support) produced by a three-dimensional object producing method;
FIG. 5 is a view illustrating a state after detachment during production by a three-dimensional object producing method; and
FIG. 6 is a view illustrating measurement of a vertical error and a horizontal error of an object after detachment.

### Mode for Carrying out the Invention

### (Hydrogel precursor liquid)

An organic-inorganic combined hydrogel precursor liquid of the present invention contains water, a polymerizable monomer, a mineral, and further contains a chelate agent and other components as needed.

### <Polymerizable monomer>

Examples of the polymerizable monomer include acrylamide, N-substituted acrylamide derivatives, N,N-disubstituted acrylamide derivatives, N-substituted methacrylamide derivatives, and N,N-disubstituted methacrylamide derivatives. One of these polymerizable monomers may be used alone or two or more of these polymerizable monomers may be used in combination. Among these polymerizable monomers, acrylamide, N,N-dimethyl acrylamide, N-isopropyl acrylamide, and N-acryloylmorpholine are preferable.

The content of the polymerizable monomer is preferably 0.5% by mass or greater but 20% by mass or less relative to the total amount of the hydrogel precursor liquid.

### <Water>

The water is not particularly limited and may be appropriately selected depending on the intended purpose. Examples of the water include pure water such as ion-exchanged water, ultrafiltrated water, reverse osmotic water, and distilled water, and ultrapure water.

Any other component such as an organic solvent may be dissolved or dispersed in the water with a view to, for example, imparting a moisture retaining property, imparting an antimicrobial activity, imparting conductivity, and adjusting hardness.

The content of the water is preferably 10% by mass or greater but 99% by mass or less, more preferably 60% by mass or greater but 98% by mass or less, and particularly preferably 70% by mass or greater but 97% by mass or less relative to the total amount of the hydrogel precursor liquid.

### <Mineral>

The mineral is not particularly limited and may be appropriately selected depending on the intended purpose as long as it is water-swellable. Examples of the mineral include a layered mineral.

The layered mineral is preferably a dispersed mineral dispersed in water in a monolayer state.

The layered mineral is not particularly limited and may be appropriately selected depending on the intended purpose. Examples of the layered mineral include a water-swellable layered clay mineral.

Examples of the water-swellable layered clay mineral include water-swellable smectite and water-swellable mica. More specific examples of the water-swellable layered clay mineral include water-swellable hectorite containing sodium as an interlayer ion, water-swellable montmorillonite, water-swellable saponite, and water-swellable synthetic mica.

The water-swellability means that a layered mineral absorbs water and undergoes volume expansion when water molecules are inserted between the layers of the layered mineral.

As the water-swellable layered clay mineral, one of the examples presented above may be used alone or two or more of the examples presented above may be used in combination, or an appropriately synthesized product or a commercially available product may be used.

Examples of the commercially available product include synthetic hectorite (LAPONITE XLG, available from Rock Wood), SWN (available from Coop Chemical Ltd.), and fluorinated hectorite SWF (available from Coop Chemical Ltd.). Among these commercially available products, synthetic hectorite is preferable.

The content of the mineral is preferably 1% by mass or greater but 40% by mass or less and more preferably 1% by mass or greater but 15% by mass or less relative to the total amount of the hydrogel precursor liquid. When the content of the mineral is 1% by mass or greater but 40% by mass or less, the hydrogel precursor liquid has an appropriate viscosity, leading to a good dischargeability through an inkjet nozzle and a good hardness of a three-dimensional object.

### <Chelate agent>

The chelate agent is contained in order to prevent a dimensional error due to, for example, liquid drooping.

The chelate agent is not particularly limited and may be appropriately selected depending on the intended purpose, so long as the chelate agent has an ion sequestering property. Examples of the chelate agent include aminocarboxylic acid-based chelate agents, phosphonic acid chelate agents, chelate metal salts, and carboxylic acid-based chelate agents. One of these chelate agents may be used alone or two or more of these chelate agents may be used in combination. Among these chelate agents, chelate agents of carboxylic acid, such as aminocarboxylic acid-based chelate agents and carboxylic acid-based chelate agents, and phosphonic acid chelate agents are preferable.

Examples of the aminocarboxylic acid-based chelate agents include ethylenediamine tetraacetic acid (EDTA), nitrilo triacetic acid (NTA), diethylenetriamine pentaacetic acid (DTPA), triethylene tetramine hexaacetic acid (TTHA), 1,2-diaminopropane-N,N,N',N'-tetraacetic acid (PDTA), N-(2-hydroxyethyl)ethylenediamine-N,N',N'-triacetic acid (HEDTA), ethylenediamine-N,N'-disuccinic acid (EDDS), dihydroxyethyl glycine (DHEG), 1,3-diamino-2-propanol-N,N,N',N'-tetraacetic acid (DPTA-OH), hydroxyimino diacetic acid (HIDA), ethylene glycol bis(β-aminoethyl ether)-N,N,N',N'-tetraacetic acid (GEDTA), dicarboxymethyl glutamic acid (CMGA), and phytic acid. One of these aminocarboxylic acid-based chelate agents may be used alone or two or more of these aminocarboxylic acid-based chelate agents may be used in combination.

Examples of the phosphonic acid chelate agent include 1-hydroxyethane-1,1-diphosphonic acid (HEDP), nitrilotrismethylene phosphonic acid (NTMP), 2-phosphobutane-1,2,4-tricarboxylic acid (PBTC), and N,N,N',N'-ethylenediamine tetrakis(methylenephosphonic acid) hydrate (EDTMP). One of these phosphonic acid chelate agents may be used alone or two or more of these phosphonic acid chelate agents may be used in combination. Among these phosphonic acid chelate agents, 1-hydroxyethane-1,1-diphosphonic acid (HEDP) is preferable.

Examples of the chelate metal salts include ethylenediamine tetraacetic acid metal salts and diethylenetriamine pentaacetic acid metal salts. One of these chelate metal salts may be used alone or two or more of these chelate metal salts may be used in combination.

Examples of the carboxylic acid-based chelate agents include gluconic acid, succinic acid, and citric acid. One of these carboxylic acid-based chelate agents may be used alone or two or more of these carboxylic acid-based chelate agents may be used in combination.

The content of the chelate agent is preferably 0.05% by mass or greater and more preferably 0.1% by mass or greater but 5% by mass or less relative to the total amount of the hydrogel precursor liquid.

The chelate agent can be measured using, for example, a pyrolysis gas chromatography (instrument name: GCMS-QP2020, available from Shimadzu Corporation).

The surface tension of the hydrogel precursor liquid is not particularly limited, may be appropriately selected depending on the intended purpose, and is preferably 20 mN/m or greater but 45 mN/m or less and more preferably 25 mN/m or greater but 34 mN/m or less.

When the surface tension of the hydrogel precursor liquid is 20 mN/m or greater, the hydrogel precursor liquid can be stably discharged during object production. When the surface tension of the hydrogel precursor liquid is 45 mN/m or less, for example, a discharging nozzle can be easily filled with the liquid during object production.

The surface tension can be measured with, for example, a surface tensiometer (automatic contact angle gauge DM-701, available from Kyowa Interface Science Co., Ltd.).

The viscosity of the hydrogel precursor liquid is not particularly limited, may be appropriately selected depending on the intended purpose, and the hydrogel precursor liquid can be appropriately used based on temperature adjustment. For example, the viscosity of the hydrogel precursor liquid is preferably 3 mPa·s or higher but 20 mPa·s or lower and more preferably 6 mPa·s or higher but 12 mPa·s or lower at 25 degrees C.

When the viscosity of the hydrogel precursor liquid is 3 mPa·s or higher, the hydrogel precursor liquid can be stably discharged during object production. When the viscosity of the hydrogel precursor liquid is 20 mPa·s or lower, the hydrogel precursor liquid can be discharged easily.

The viscosity can be measured with, for example, a rotational viscometer (VISCOMATE VM-150III, available from Toki Sangyo Co., Ltd.) in an environment of 25 degrees C.

### <Other components>

The other components are not particularly limited and may be appropriately selected depending on the intended purpose. Examples of the other components include a stabilizing agent, a surface treating agent, a polymerization initiator, a colorant, a viscosity modifier, a tackifier, an antioxidant, an age resister, a cross-linking promoter, an ultraviolet absorber, a plasticizer, an antiseptic, and a dispersant.

### -Stabilizing agent-

The stabilizing agent is used in order to stabilize dispersion of the mineral and maintain a sol-state. Furthermore, in an inkjet method, the stabilizing agent is used in order to stabilize properties as a liquid.

Examples of the stabilizing agent include high-concentration phosphates, glycol, and nonionic surfactants.

### -Surface treating agent-

Examples of the surface treating agent include polyester resins, polyvinyl acetate resins, silicone resins, coumarone resins, fatty acid esters, glycerides, and waxes.

### (Material set for producing three-dimensional object)

As a material set for producing a three-dimensional object, there are a mode A that includes a hydrogel precursor liquid containing water, a mineral, and a polymerizable monomer, and a chelate agent-containing liquid, and a mode B that includes a first hydrogel precursor liquid containing water, a polymerizable monomer, a mineral, and a chelate agent and a second hydrogel precursor liquid compositionally different from the first hydrogel precursor liquid.

As the water, the polymerizable monomer, the mineral, and the chelate agent in the mode A and the mode B, the same substances as used as the water, the polymerizable monomer, the mineral, and the chelate agent in the hydrogel precursor liquid of the present invention can be used.

### -Mode A-

The hydrogel precursor liquid in the mode A contains water, a mineral, and a polymerizable monomer, further contains other components as needed, and does not contain a chelate agent.

The chelate agent-containing liquid in the mode A contains a chelate agent and further contains other components as needed.

As the other components, the same substances as used as the other components in the hydrogel precursor liquid of the present invention can be used.

### -Mode B-

The first hydrogel precursor liquid in the mode B contains water, a polymerizable monomer, a mineral, and a chelate agent, and preferably further contains other components as needed.

The second hydrogel precursor liquid in the mode B is compositionally different from the first hydrogel precursor liquid.

The first hydrogel precursor liquid and the second hydrogel precursor liquid contain the same components, but the blending ratio of the components in the first hydrogel precursor liquid and the blending ratio of the components in the second hydrogel precursor liquid are different from each other.

### (Three-dimensional object producing method and three-dimensional object producing apparatus)

A three-dimensional object producing method of the present invention is not particularly limited and may be appropriately selected depending on the intended purpose. Examples of the three-dimensional object producing method include a first mode including a step of injecting a hydrogel precursor liquid containing water, a polymerizable monomer, and a chelate agent into a mold, and a second mode repeating a plurality of times a first step of applying a hydrogel precursor liquid containing water and a polymerizable monomer and a chelate agent-containing liquid to form a film.

The three-dimensional object producing method of the present invention is based on the following finding. Existing three-dimensional object producing methods need a liquid-state photo-curable resin to be stored in a large amount and hence need upsizing of the apparatus. Moreover, the existing methods need management of, for example, temperature in order to stabilize the quality of the liquid-state photo-curable resin.

A three-dimensional object producing apparatus used in the present invention includes a storing unit configured to store a hydrogel precursor liquid containing water and a polymerizable monomer, a storing unit configured to store a chelate agent-containing liquid, at least two applying units configured to apply the hydrogel precursor liquid and the chelate agent-containing liquid, and an irradiating unit configured for ultraviolet irradiation, and can be suitably used for the second mode of the three-dimensional object producing method of the present invention.

### <First mode of three-dimensional object producing method>

The first mode of the three-dimensional object producing method includes a step of injecting a hydrogel precursor liquid containing water, a polymerizable monomer, a water-swellable mineral and a chelate agent into a mold, and further includes other steps as needed.

### «Mold»

The mold is not particularly limited and may be appropriately selected depending on the intended purpose, so long as the mold is formed of a material that is not permeated by the hydrogel precursor liquid. It is preferable to use a mold from which a liquid does not leak, because the hydrogel precursor liquid is present in the form of a liquid.

The mold can be produced using, for example, mechanical polishing and cutting. The mold can also be produced with a known inkjet stereolithography apparatus (for example, a 3D printer, apparatus name: AGILISTA, available from Keyence Corporation).

The method for injection into the mold is not particularly limited and may be appropriately selected depending on the intended purpose. Examples of the method include a pouring method, a dispenser method, a spray method, and an inkjet method. Known apparatuses can be suitably used for performing these methods.

### <Second mode of three-dimensional object producing method and three-dimensional object producing apparatus>

The second mode of the three-dimensional object producing method of the present invention includes a first step of applying a hydrogel precursor liquid and a chelate agent-containing liquid to form a film, and preferably includes a second step of curing the film formed in the first step, and produces a three-dimensional object by repeating the first step and the second step and by including a third step of forming a support and other steps as needed.

The order of performing the first step and second step, and the third step is not particularly limited. The third step may be performed after the first step and second step, or the third step may be performed before the first step and second step. Of these orders, the order of performing the third step before the first step and second step is preferable, because a support can be formed first.

The second mode of the three-dimensional object producing method is intended to repeat the steps a plurality of times. The number of times of repetition is not to be flatly determined because the number of times of repetition is different depending on, for example, the size, shape, and structure of the three-dimensional object to be produced. It is preferable to repeat laminating layers a number of times corresponding to the height of the three-dimensional object to be produced, so long as the average thickness per layer is in a range in which accurate object production without peeling is available, specifically 10 micrometers or greater but 50 micrometers or less.

The three-dimensional object producing apparatus used in the present invention includes a storing unit configured to store a hydrogel precursor liquid containing a polymerizable monomer, a storing unit configured to store a chelate agent-containing liquid free of the polymerizable monomer but containing a chelate agent, at least two applying units configured to apply the hydrogel precursor liquid and the chelate agent-containing liquid, and an irradiating unit configured for ultraviolet irradiation, preferably includes a first unit configured to apply the hydrogel precursor liquid and the chelate agent-containing liquid to form a film and a second unit configured to cure the film, and further includes other units as needed.

The storing units are not particularly limited and may be appropriately selected depending on the intended purpose, so long as the storing units are capable of storing the hydrogel precursor liquid and the chelate agent-containing liquid.

As the two applying units, it is preferable to use inkjet heads.

### <<First step and first unit>>

The first step is a step of applying a hydrogel precursor liquid containing a polymerizable monomer and a chelate agent-containing liquid free of the polymerizable monomer but containing a chelate agent to form a film, and can be performed by the first unit.

The hydrogel precursor liquid contains water and a polymerizable monomer, and preferably is free of a chelate agent.

With a chelate agent contained in the chelate agent-containing liquid, a mixture liquid obtained when the hydrogel precursor liquid and the chelate agent-containing liquid are mixed with each other undergoes viscosity thickening again. This can prevent a dimensional error due to, for example, liquid drooping.

In the first step, the hydrogel precursor liquid and the chelate agent-containing liquid are discharged to the same position and mixed with each other, to form a formation pattern. Unlike a case where an object pattern is formed of only the hydrogel precursor liquid, use of the chelate agent-containing liquid allows the formation pattern to undergo viscosity thickening and lose fluidity rapidly. Hence, liquid drooping is not likely to occur from the coated film, and the formation accuracy of the pattern can be significantly improved. This makes it possible to produce a complicated, accurate three-dimensional object having an excellent compressive strength.

The first step preferably includes a hydrogel precursor liquid applying process of applying the hydrogel precursor liquid, and a chelate agent-containing liquid applying process of applying the chelate agent-containing liquid, and can be performed by a hydrogel precursor liquid applying member and a chelate agent-containing liquid applying member. It is preferable that the hydrogel precursor liquid applying member and the chelate agent-containing liquid applying member be different members from each other.

The method for and the unit configured for applying the hydrogel precursor liquid and the chelate agent-containing liquid as the first step (the hydrogel precursor liquid applying process and the chelate agent-containing liquid applying process) and the first unit (the hydrogel precursor liquid applying member and the chelate agent-containing liquid applying member) are not particularly limited and may be appropriately selected depending on the intended purpose, so long as the method and the unit are of the type that is capable of coating a liquid droplet on an intended position with an appropriate accuracy. Examples of the method and the unit include a dispenser method, a spray method, and an inkjet method. Known apparatuses can be suitably used for performing these methods.

Among these methods, the dispenser method has excellent liquid droplet quantitativity, but has a small coating coverage. The spray method can form a minute jet of the materials easily and has a wide coating coverage and excellent coatability, but has a poor liquid droplet quantitativity and causes scattering due to a spray current. Hence, in the present invention, the inkjet method is particularly preferable. The inkjet method is preferable because the inkjet method is better than the spray method in liquid droplet quantitativity, can obtain a greater coating coverage than can be obtained by the dispenser method, and can form a complicated three-dimensional shape with a good accuracy efficiently.

In the case of the inkjet method, nozzles capable of discharging the hydrogel precursor liquid are provided. Nozzles of a known inkjet printer can be favorably used as the nozzles.

### <<<Second step and second unit>>>

The second step is a step of curing the film formed in the first step, and can be performed by the second unit.

Examples of the unit as the second unit configured to cure a film include an ultraviolet (UV) irradiation lamp, and an electron beam. The unit configured to cure a film preferably includes a mechanism configured to remove ozone.

Examples of the kind of the ultraviolet (UV) irradiation lamp include a highpressure mercury lamp, an ultrahigh-pressure mercury lamp, metal halides, and an ultraviolet light emitting diode (UV-LED).

The ultrahigh-pressure mercury lamp is a point light source. However, a Deep UV type ultrahigh-pressure mercury lamp combined with an optical system for a higher light utilization efficiency is capable of irradiation in a short-wavelength range.

The metal halides having a wide wavelength range are effective for colored materials, and are formed of halides of metals such as Pb, Sn, and Fe, which can be selected depending on the absorption spectrum of a polymerization initiator. The lamp used for curing is not particularly limited and may be appropriately selected depending on the intended purpose. For example, commercially available lamps such as H LAMP, D LAMP, or V LAMP available from Fusion Systems Inc. can be used.

The emission wavelength of the ultraviolet light emitting diode is not particularly limited and may be appropriately selected depending on the intended purpose. Emission wavelengths of common ultraviolet light emitting diodes are 365 nm, 375 nm, 385 nm, 395 nm, and 405 nm. Considering chromatic influences to a three-dimensional object, shorter wavelength emission is more advantageous for a greater absorption by the polymerization initiator. Among these ultraviolet (UV) irradiation lamps, an ultraviolet light emitting diode (UV-LED) with low heat generation is preferable, considering application to a three-dimensional object of the present invention formed of a hydrogel that is susceptible to thermal energy.

The film after cured is an organic-inorganic combined hydrogel formed by water and components soluble in the water being contained in a three-dimensional network structure formed by a water-soluble polymer and a mineral being combined with each other.

The organic-inorganic combined hydrogel has an improved extensibility and can be integrally peeled without being torn. This significantly simplifies any treatment after object production.

The rubber hardness of the organic-inorganic combined hydrogel is preferably 6 or higher but 60 or lower and more preferably 8 or higher but 20 or lower. When the rubber hardness of the organic-inorganic combined hydrogel is 6 or higher, shape collapse during object production can be prevented. When the rubber hardness of the organic-inorganic combined hydrogel is 60 or lower, cracking upon detachment after object production can be prevented.

The rubber hardness can be measured with, for example, a durometer (available from Teclock Corporation, GS-718N).

### «Third step and third unit»

The third step is a step of applying a support forming liquid containing at least a polymerizable monomer to a position different from the position to which the hydrogel precursor liquid and the chelate agent-containing liquid are applied to form a support, and can be performed by a third unit.

"The position different from the position to which the hydrogel precursor liquid and the chelate agent-containing liquid are applied" means that the position to which the support forming liquid is applied and the position to which a first liquid and the chelate agent-containing liquid are applied do not overlap. The position to which the support forming liquid is applied and the position to which the hydrogel precursor liquid and the chelate agent-containing liquid are applied may adjoin each other.

As the method for and the unit configured for applying the support forming liquid, the same method and unit as used for applying the hydrogel precursor liquid and the chelate agent-containing liquid can be used.

### <<<Support forming liquid>>>

The support forming liquid contains at least a polymerizable monomer, and further contains a polymerization initiator, a colorant, and other components as needed. The support forming liquid is also referred to as "material for a hard shaped body".

The polymerizable monomer is not particularly limited and may be appropriately selected depending on the intended purpose, so long as the polymerizable monomer is a compound that cures in response to, for example, active energy ray irradiation or heating. Examples of the polymerizable monomer include an active-energy-ray-curable compound, an active-energy-ray-curable prepolymer, an emulsion-type photo-curable resin, and a thermosetting compound. Among these polymerizable monomers, materials that are liquid at normal temperature are preferable in terms of preventing nozzle clogging.

The content of the polymerizable monomer is preferably 60% by mass or greater but 100% by mass or less, more preferably 80% by mass or greater but 100% by mass or less, and particularly preferably 90% by mass or greater but 100% by mass or less relative to the total amount of the support forming liquid.

### -Polymerization initiator-

As the polymerization initiator, an arbitrary substance that produces radicals in response to irradiation of light (particularly, an ultraviolet ray having a wavelength of 220 nm or longer but 400 nm or shorter) can be used.

The surface tension of the support forming liquid may be the same as the surface tension of the hydrogel precursor liquid.

The viscosity of the support forming liquid may be the same as the viscosity of the hydrogel precursor liquid.

### <<Fourth step (smoothing unit) and fourth unit>>

The fourth step (smoothing unit) is a step of smoothing the film cured in the second step, and can be performed by a fourth unit.

The fourth unit is not particularly limited and may be appropriately selected depending on the intended purpose. Examples of the fourth unit include a roller and a blade.

### -Roller-

For example, the shape, structure, size, and material of the roller are not particularly limited and may be appropriately selected depending on the intended purpose. Examples of the shape of the roller include a circular-columnar solid body and a hollow cylindrical shape. Examples of the structure of the roller include a single-layer structure and a laminated structure. The size of the roller may be appropriately selected depending on, for example, the size of the three-dimensional object. Examples of the material of the roller include a resin, a rubber, a metal, and any combination of these materials.

Examples of the roller include a rubber roller including a cored bar and a rubber layer over the cored bar, a rubber roller formed only of a rubber without a cored bar, a foamed roller including a core material and a foamed layer formed over the outer circumference of the core material, and a metal roller.

### <<Other steps and other units>>

The other steps are not particularly limited and may be appropriately selected depending on the intended purpose. Examples of the other steps include a detaching step, a discharging stabilizing step, an object washing step, and an object polishing step. These steps can be performed by, for example, a discharging stabilizing unit, an object washing unit, and an object polishing unit.

### «<Detaching step»>

The detaching step is a step of detaching a portion formed of a three-dimensional object, which is a hydrogel containing water as a main component and produced from the hydrogel precursor liquid, and a portion formed of a polymer formed from the polymerizable monomer from each other, and can be performed by a detaching unit.

The detaching unit is not particularly limited and may be appropriately selected depending on the intended purpose. Examples of the detaching unit include various detaching devices.

### «<Discharging stabilizing step and discharging stabilizing unit>»

In the case of using an inkjet head as a unit configured to discharge a liquid, dryness of the nozzles during non-discharging is a major problem against the stabilizing operation.

Hence, when continuous discharging from an inkjet head is not to be performed for a long time, the discharging stabilizing step and the discharging stabilizing unit can maintain a discharging stabilized state for a long time, by (1) covering (capping) the discharging port with a member having a shape covering at least the leading end of the head to prevent the leading end of the discharging port from being dried, (2) removing a coating film formed by the internal liquid present near the discharging port being thickened in viscosity due to drying or being dried by a sucking action, or (3) wiping the discharging port, or the discharging port and the surroundings.

These things matter significantly for a step of producing a three-dimensional object that needs long-time continuous discharging longer than or equal to 24 hours, particularly for a case where a liquid containing a low-boiling-point solvent such as water is used for object production using a soft material.

As described above, in the three-dimensional object producing method of the present invention, the liquids are applied in a manner to enable images to be formed layer by layer, by being discharged through small holes by, for example, an inkjet method or a dispenser method, and the hydrogel precursor liquid and the chelate agent-containing liquid before curing mix with each other in a state of being discharged to the same position, while the portion of the support forming liquid contacting the hydrogel precursor liquid and the chelate agent-containing liquid is clearly separated and in an immiscible, incompatible state.

In the three-dimensional object producing method of the present invention, the hydrogel precursor liquid and the chelate agent-containing liquid that are in the state of being incompatible with the support forming liquid will have a clear boundary with the support forming liquid after photo curing. Furthermore, a hardness difference between the obtained object and the support improves detachability. This improves the surface smoothness of the object and makes it possible to skip or significantly shorten the polishing step after three-dimensional object production.

A specific embodiment of the second mode of the three-dimensional object producing method and the three-dimensional object producing apparatus of the present invention will be described below.

A three-dimensional object, which is a soft hydrogel, can be obtained using a hydrogel precursor liquid containing a hydrogel precursor and a chelate agent-containing liquid free of a hydrogel precursor but containing a chelate agent (hereinafter may also be referred to as "liquid materials for a soft shaped body") and using a support forming liquid containing a polymerizable monomer (hereinafter may also be referred to as "liquid material for a hard shaped body").

First, surface data or solid data representing a three-dimensional shape designed by three-dimensional CAD or a three-dimensional shape scanned with a three-dimensional scanner or a digitizer is converted into a STL format and input to the three-dimensional object producing apparatus.

Based on the input data, an object producing orientation for producing a three-dimensional shape to be produced is determined. The object producing orientation is not particularly limited, and, typically, an orientation that has the smallest size in the Z-direction (height direction) is selected.

After the object producing orientation is determined, the projected areas of the three-dimensional shape on an X-Y plane, an X-Z plane, and a Y-Z plane are obtained. In order to reinforce the obtained block shape, the surfaces of the block shape except for the top surface in the X-Y plane are shifted outward by an appropriate distance. The distance by which the surfaces are shifted is not particularly limited and is different depending on the shape, the size, and the liquid materials used, but is about 1 mm or longer but 10 mm or shorter. In this way, a block shape enclosing the shape to be produced (with the top surface opened) is specified.

This block shape is sliced in the Z-direction at intervals determined by the thickness of one layer. The thickness of one layer is different depending on the materials used and is not to be defined flatly, but is preferably 10 micrometers or greater but 50 micrometers or less.

When there is one three-dimensional object to be produced, this block shape is disposed in the center of a Z stage (which is an object placing table that is configured to lift down by a distance corresponding to one layer every time one layer is formed). In the case of producing a plurality of objects simultaneously, block shapes are disposed on the Z stage, or alternatively, the block shapes may be stacked one upon another. These operations of block shaping, slice data (contour data) generation, and Z stage positioning may be automated with designation of the liquid materials to be used.

Next, in an object producing step, the position to which the liquid materials for a soft shaped body are discharged and the position to which the liquid material for a hard shaped body is discharged are controlled by inside-outside judgment based on the outermost contour line in the slice data (for judging which of the liquid materials for a soft shaped body and the liquid material for a hard shaped body are/is to be discharged to a position on the contour line).

Further, high-speed object production is available by positioning an active energy ray irradiator to adjoin an inkjet head configured to discharge the liquid materials for a soft shaped body.

Furthermore, in order to smooth a layer formed for producing a three-dimensional object, a smoothing treatment is performed immediately after a curing treatment is performed.

The smoothing treatment is for smoothing the surface of a cured film using a smoothing member such as a roller and a blade. This improves the accuracy of each layer, and enables accurate production of a three-dimensional object as a whole.

Here, in order to reduce the layer lamination time and improve smoothness of the layers, it is preferable to position the smoothing member to adjoin the ultraviolet ray irradiator.

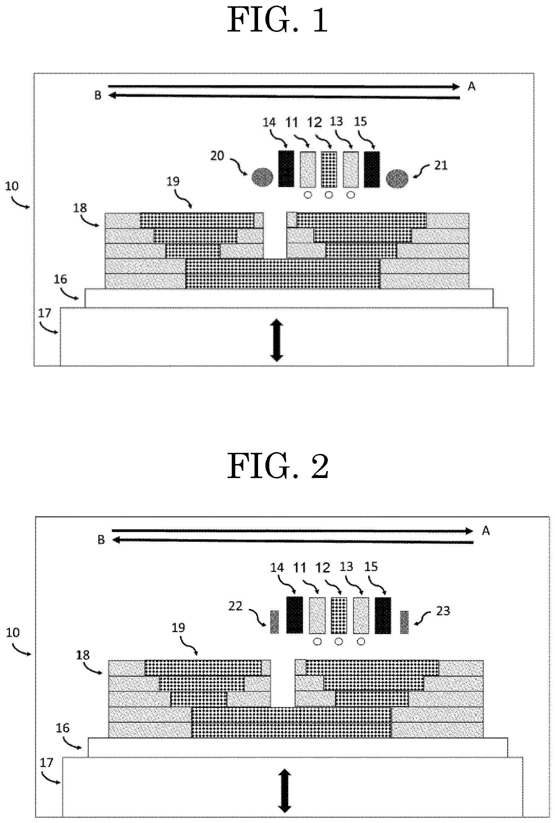

FIG. 1 is a schematic view illustrating an example of an object producing step in the three-dimensional object producing method of the present invention using the three-dimensional object producing apparatus.

Using a head unit in which inkjet heads are arranged, a three-dimensional object producing apparatus 10 is configured to laminate layers by discharging the hydrogel precursor liquid from a liquid material discharging head unit 11 for an object, the chelate agent-containing liquid from a liquid material discharging head unit 12 for an object, and the support forming liquid from an ink discharging head unit 13 for a support, and curing the hydrogel precursor liquid and the chelate agent-containing liquid with adjoining ultraviolet ray irradiators 14 and 15.

That is, the support forming liquid is discharged from the inkjet head (liquid material discharging head unit 13 for a support) and solidified to form a first support layer including a reservoir. The hydrogel precursor liquid containing a hydrogel precursor and the chelate agent-containing liquid free of a hydrogel precursor but containing a chelate agent are discharged into the reservoir of the first support layer from the inkjet heads (liquid material discharging head units 11 and 12 for an object), and a film formed of the hydrogel precursor liquid and the chelate agent-containing liquid is irradiated with an active energy ray to be cured. Furthermore, a smoothing treatment is applied to the cured film by smoothing members 20 and 21, to form a first three-dimensional object layer. These steps are repeated sequentially, to produce a three-dimensional laminated object 19 having a three-dimensionally laminated structure.

In the three-dimensional object producing apparatus 10 of this type, the ultraviolet ray irradiators 14 and 15 are used in moving in the directions of both of the arrows A and B. The surface of a laminated layer of the material for a hard shaped body is smoothed by the heat generated along with the ultraviolet ray irradiation. As a result, the dimensional stability of the object can be improved.

Moreover, in order to maintain the liquid material discharging head units 11, 12, and 13 and the ultraviolet ray irradiators 14 and 15 at a constant gap from an object 19 and a support 18, layer lamination is performed while a stage 17 is lifted down in accordance with the number of times of layer lamination.

FIG. 2 is a schematic view illustrating another example of an object producing step in the three-dimensional object producing method of the present invention using the three-dimensional object producing apparatus. Specifically, the smoothing members of FIG. 1 are changed to members having a blade shape. This is more effective than the members having a roller shape used in FIG. 1, for a case where the surface of the object is to be scraped for smoothing.

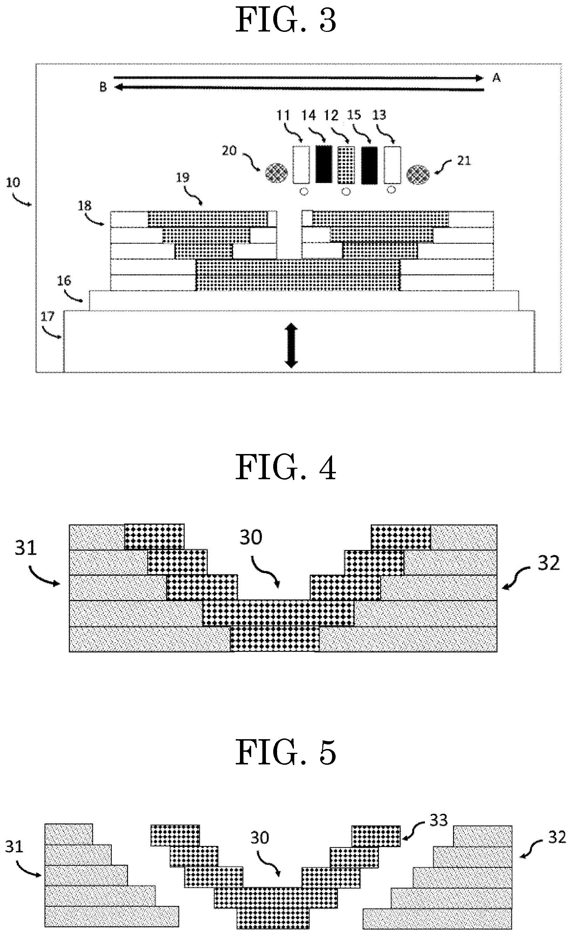

FIG. 3 is a schematic view illustrating another example of a three-dimensional object producing step configured to be capable of improving the smoothness of each layer better than in FIG. 1. The basic step is the same as in FIG. 1, but the difference is that the ultraviolet ray irradiator 14 is arranged between the liquid material discharging head 11 for an object and the liquid material discharging head 12 for an object and the ultraviolet ray irradiator 15 is arranged between the liquid material discharging head 12 for an object and the liquid material discharging head 13 for a support.

In the three-dimensional object producing apparatus 10 of this type, the ultraviolet ray irradiators 14 and 15 are used in moving in the directions of both of the arrows A and B. The surface of a laminated layer of the liquid material for a hard shaped body is smoothed by the heat generated along with the ultraviolet ray irradiation. As a result, the dimensional stability of the object is improved.

The three-dimensional object producing apparatus 10 may additionally include, for example, a liquid collecting mechanism, a maintenance unit (maintenance mechanism), and a recycling mechanism. The three-dimensional object producing apparatus may also include a blade configured to remove a liquid material that has adhered to a nozzle surface and a mechanism configured to detect a nozzle that has failed in discharging. Furthermore, it is also preferable to control an environmental temperature in the three-dimensional object producing apparatus during production of a three-dimensional object.

With the maintenance unit (maintenance mechanism), the liquid materials for a soft shaped body and the liquid material for a hard shaped body can be prevented from hardening when collected by the liquid collecting mechanism.

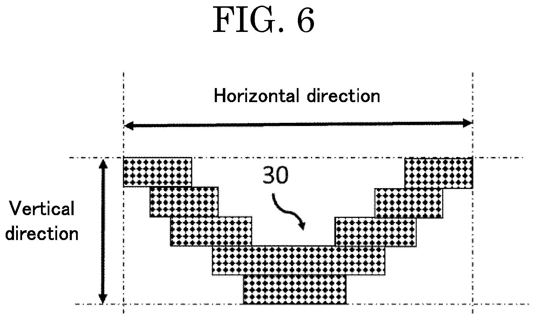

The supports 31 and 32 are physically detached and removed from a surface 33 of the object finally produced as a three-dimensional object as illustrated in FIG. 4 and FIG. 5, and a laminated object as illustrated in FIG. 6 can be obtained.

### (Three-dimensional object)

A three-dimensional object of the present invention is a three-dimensional object, which is a hydrogel containing water as a main component, and contains a chelate agent, and further contains a water-soluble polymer and other components as needed.

It is preferable that the three-dimensional object of the present invention be a laminated object produced by a laminated object manufacturing method.

The three-dimensional object of the present invention can be suitably used as an organ model.

The compressive strength of the three-dimensional object at 70% compression is preferably 0.5 MPa or higher and more preferably 1.0 MPa or higher. When the compressive strength of the three-dimensional object at 70% compression is 0.5 MPa or higher, the three-dimensional object can have a high strength. The compressive strength at 70% compression can be measured with an instrument obtained by mounting a dynamic ultramicro hardness meter (DUH-W201S, available from Shimadzu Corporation) with software for a microcompression tester (MCT-W, available from Shimadzu Corporation).

### <Hydrogel>

The hydrogel is an organic-inorganic combined hydrogel, and is not particularly limited and may be appropriately selected depending on the intended purpose, so long as the hydrogel contains water as a main component.

The hydrogel is preferably a gel formed by water being absorbed into a network structure of a polymer.

The content of water in the hydrogel is preferably 10% by mass or greater but 99% by mass or less, more preferably 60% by mass or greater but 98% by mass or less, and particularly preferably 70% by mass or greater but 97% by mass or less relative to the total amount of the hydrogel. In a case where the hydrogel undergoes phase change in response to an external stimulus and swells or shrinks, the content of water refers to a content of water before swelling or shrinking.

### <Chelate agent>

As the chelate agent, the same substances as presented as the chelate agent in the hydrogel precursor liquid of the present invention can be used.

The content of the chelate agent is preferably 0.05% by mass or greater and more preferably 0.1% by mass or greater but 5% by mass or less relative to the total amount of the three-dimensional object.

The content of the chelate agent can be measured using, for example, a pyrolysis gas chromatography (instrument name: GCMS-QP2020, available from Shimadzu Corporation).

### <Water-soluble polymer>

As the water-soluble polymer, a polymer containing, for example, an amide group, an amino group, a hydroxyl group, a tetramethyl ammonium group, a silanol group, or an epoxy group can be obtained by polymerizing the polymerizable monomer in the hydrogel precursor liquid of the present invention by active energy ray irradiation. With the water-soluble polymer contained, the hydrogel can have an improved strength.

### Examples

The present invention will be described more specifically below by way of Examples. The present invention should not be construed as being limited to these Examples.

### (Example 1)

### <Preparation of hydrogel precursor liquid>

Hereinafter, ion-exchanged water subjected to vacuum degassing for 10 minutes will be referred to as pure water.

Next, to pure water (20 parts by mass) under stirring, synthetic hectorite (LAPONITE XLG, available from Rock Wood) having a composition [Mg_{5.34}Li_{0.66}Si₈O₂₀(OH)₄]⁻_{0.66}Na⁺_{0.66} (0.8 parts by mass) was added little by little as a layered clay mineral and stirred, to produce a dispersion liquid.

Next, to the dispersion liquid, N,N-dimethylacrylamide (available from Wako Pure Chemical Industries., Ltd.) (4 parts by mass) having been passed through an activated alumina column for removal of a polymerization inhibitor was added as a polymerizable monomer. Next, sodium dodecyl sulfate (available from Wako Pure Chemical Industries, Ltd.) (0.01 parts by mass) was added as a surfactant and mixed.

Next, 1-hydroxyethane-1,1-diphosphonic acid (0.5 parts by mass) was added as a chelate agent.

Next, to the obtained mixture liquid under cooling in an ice bath, tetramethylethylene diamine (available from Wako Pure Chemical Industries, Ltd.) (0.005 parts by mass) was added as a polymerization initiator.

Further, an aqueous solution (2 parts by mass) obtained by dissolving sodium peroxo disulfate (available from Wako Pure Chemical Industries, Ltd.) (0.04 parts by mass) in pure water (1.96 parts by mass) was added as a polymerization initiator liquid, stirred and mixed, and subsequently subjected to vacuum degassing for 10 minutes, to obtain a homogeneous hydrogel precursor liquid. The composition is presented in Table 1.

### <Three-dimensional object production>

The obtained hydrogel precursor liquid for gel was injected into a mold described below, left to stand still in an environment of 25 degrees C for 20 hours, and taken out from the mold, to obtain a three-dimensional object, which was a hydrogel containing water as a main component.

### <Production of mold>

With an inkjet stereolithography apparatus having an apparatus name AGILISTA (available from Keyence Corporation), a mold for a human liver was produced by applying three-dimensional model data for a human liver.

The content of the chelate agent in the obtained three-dimensional object was measured using a pyrolysis gas chromatography (instrument name: GCMS-QP2020, available from Shimadzu Corporation). The result is presented in Table 3.

"Object production accuracy" and "compressive strength" of the obtained three-dimensional object were evaluated in the manners described below.

### <Object production accuracy>

The volume of the obtained three-dimensional object was measured with a laser scanner, to measure discrepancy from the CAD data dimensions, which were seen to be 100%, and evaluate the object production accuracy. A value closer to 100% indicates a smaller discrepancy. The result is presented in Table 3.

### <Compressive strength at 70% compression>

The compressive strength (MPa) of the obtained three-dimensional object at 70% compression was measured with an instrument obtained by mounting a dynamic ultramicro hardness meter (DUH-W201S, available from Shimadzu Corporation) with software for a microcompression tester (MCT-W, available from Shimadzu Corporation). The result is presented in Table 3.

### (Example 2)

A three-dimensional object was obtained in the same manner as in Example 1, except that unlike in Example 1, the chelate agent was changed from 1-hydroxyethane-1,1-diphosphonic acid to succinic acid (0.5 parts by mass).

"Object production accuracy" and "compressive strength" of the obtained three-dimensional object were evaluated in the same manners as in Example 1. The results are presented in Table 3.

### (Example 3)

A three-dimensional object was obtained in the same manner as in Example 1, except that unlike in Example 1, 1-hydroxyethane-1,1-diphosphonic acid (0.5 parts by mass) was changed to 1-hydroxyethane-1,1-diphosphonic acid (0.25 parts by mass).

"Object production accuracy" and "compressive strength" of the obtained three-dimensional object were evaluated in the same manners as in Example 1. The results are presented in Table 3.

### (Example 4)

A three-dimensional object was obtained in the same manner as in Example 1, except that unlike in Example 1, 1-hydroxyethane-1,1-diphosphonic acid (0.5 parts by mass) was changed to 1-hydroxyethane-1,1-diphosphonic acid (0.1 parts by mass).

"Object production accuracy" and "compressive strength" of the obtained three-dimensional object were evaluated in the same manners as in Example 1. The results are presented in Table 3.

### (Comparative Example 1)

A three-dimensional object was obtained in the same manner as in Example 1, except that unlike in Example 1, 1-hydroxyethane-1,1-diphosphonic acid serving as a chelate agent was not added. The composition is presented in Table 1.

"Object production accuracy" and "compressive strength" of the obtained three-dimensional object were evaluated in the same manners as in Example 1. The results are presented in Table 3.

**Table 1**

| | | Ex. | | | | Comp. Ex. |
|---|---|---|---|---|---|---|
| | | 1 | 2 | 3 | 4 | 1 |
| Initiator liquid | Pure water | 1.96 | 1.96 | 1.96 | 1.96 | 1.96 |
| | Sodium peroxo disulfate | 0.04 | 0.04 | 0.04 | 0.04 | 0.04 |
| Polymerizable monomer | N,N-dimethylacrylamide | 4 | 4 | 4 | 4 | 4 |
| Mineral | Synthetic hectorite | 0.8 | 0.8 | 0.8 | 0.8 | 0.8 |
| Chelate agent | 1-Hydroxyethane-1,1-diphosphonic acid | 0.5 | - | 0.25 | 0.1 | - |
| | Succinic acid | - | 0.5 | - | - | - |
| Polymerization initiator | Tetramethylethylene diamine | 0.005 | 0.005 | 0.005 | 0.005 | 0.005 |
| Pure water | | 20 | 20 | 20 | 20 | 20 |
| Total (part by mass) | | 27.305 | 27.305 | 27.055 | 26.905 | 26.805 |
| Content of chelate agent (% by mass) | | 1.83 | 1.83 | 0.92 | 0.37 | - |

### (Example 5)

### <Preparation of hydrogel precursor liquid>

To pure water (20 parts by mass) under stirring, synthetic hectorite (LAPONITE XLG, available from Rock Wood) having a composition [Mg_{5.34}Li_{0.66}Si₈O₂₀(OH)₄]-_{0.66}Na⁺_{0.66} (0.8 parts by mass) was added little by little as a layered clay mineral and stirred, to produce a dispersion liquid.

Next, to the dispersion liquid, N,N-dimethylacrylamide (available from Wako Pure Chemical Industries., Ltd.) (4 parts by mass) having been passed through an activated alumina column for removal of a polymerization inhibitor was added as a polymerizable monomer. Next, sodium dodecyl sulfate (available from Wako Pure Chemical Industries, Ltd.) (0.01 parts by mass) was added as a surfactant and mixed.

Next, 1-hydroxycyclohexylphenyl ketone (available from BASF, product name: IRGACURE 184) (0.015 parts by mass) was added as a polymerization initiator and mixed.

Next, to the obtained mixture liquid under cooling in an ice bath, tetramethylethylene diamine (available from Wako Pure Chemical Industries, Ltd.) (0.01 parts by mass) was added, and mixed and stirred, to obtain a hydrogel precursor liquid for hydrogel production.

### <Preparation of chelate agent-containing liquid>

To pure water (20 parts by mass), 1-hydroxyethane-1,1-diphosphonic acid (0.5 parts by mass) was added as a chelate agent and stirred.

Next, sodium dodecyl sulfate (available from Wako Pure Chemical Industries, Ltd.) (0.01 parts by mass) was added as a surfactant and mixed, to obtain a chelate agent-containing liquid.

### <Preparation of support forming liquid>

A total of 105 parts by mass, namely, urethane acrylate (available from Mitsubishi Rayon Co., Ltd., product name: DIABEAM UK6038) (10 parts by mass), neopentyl glycol hydroxypivalic acid ester di(meth)acrylate (available from Nippon Kayaku Co., Ltd., product name: KAYARAD MANDA) (90 parts by mass) as a polymerizable monomer, 1-hydroxycyclohexylphenyl ketone (available from BASF, product name: IRGACURE 184) (3 parts by mass) as a polymerization initiator, and a blue pigment (available from Toyo Ink Co., Ltd., product name: LIONOL BLUE 7400G) (2 parts by mass) as a colorant were subjected to dispersion treatment using a homogenizer (available from Koki Holdings Co., Ltd., HG30) at a rotation speed of 2,000 rpm until a homogeneous mixture was obtained. Subsequently, the mixture was filtrated to remove, for example, impurities, and finally subjected to vacuum degassing for 10 minutes, to obtain a homogeneous support forming liquid.

The compositions of the hydrogel precursor liquid, the chelate agent-containing liquid, and the support forming liquid are presented in Table 2.

### <Production of three-dimensional object>

The hydrogel precursor liquid, the chelate agent-containing liquid, and the support forming liquid were filled in three tanks leading to inkjet heads (available from Ricoh Industry Company, Ltd., GEN4) of the three-dimensional object producing apparatus 10 illustrated in FIG. 1, and discharged from the inkjet heads, to form a film. The hydrogel precursor liquid and the chelate agent-containing liquid were discharged to the same position, and the support forming liquid was discharged to a position different from the position to which the hydrogel precursor liquid and the chelate agent-containing liquid were discharged.

Next, with the ultraviolet ray irradiators (available from Ushio Inc., SPOT CURE SP5-250DB) 14 and 15, the film was irradiated with a light volume of 350 mJ/cm² to be cured. Subsequently, a smoothing treatment was applied to the cured film with rollers 20 and 21. This inkjet film formation was repeated layer by layer, to produce a desired three-dimensional object.

As the rollers, metal rollers formed of an aluminum alloy surface-treated with anodized aluminum and having a diameter of 25 mm were used.

The structure formed of the third liquid functioned as a support member configured to support an object to be produced by forming films of the first and second liquids.

Finally, the structure formed of the third liquid and the object produced by forming films of the first and second liquids were physically detached from each other, to obtain a desired three-dimensional object.

**"Object** production accuracy" and "compressive strength" of the obtained three-dimensional object were evaluated in the same manners as in Example 1. The content of the chelate agent in the obtained three-dimensional object was measured in the same manner as in Example 1. The results are presented in Table 3.

### (Example 6)

The hydrogel precursor liquid and the chelate agent-containing liquid described in Example 5 were filled in a static mixer-type, 2-liquid-stirring-type dispenser (available from Heishin Ltd.), and delivered at a mass ratio of 1:1 under stirring, to form a film having a desired object production pattern over a substrate for one layer.

Next, with the ultraviolet ray irradiators (available from Ushio Inc., SPOT CURE SP5-250DB) 14 and 15, the film was irradiated with a light volume of 350 mJ/cm² to be cured.

This sequence was repeated, to obtain a desired three-dimensional object. "Object production accuracy" and "compressive strength" of the obtained three-dimensional object were evaluated in the same manners as in Example 1. The content of the chelate agent in the obtained three-dimensional object was measured in the same manner as in Example 1. The results are presented in Table 3.

### (Comparative Example 2)

A three-dimensional object was obtained in the same manner as in Example 5, except that unlike in Example 5, 1-hydroxyethane-1,1-diphosphonic acid serving as a chelate agent was not added. The compositions of the hydrogel precursor liquid, the chelate agent-containing liquid, and the support forming liquid are presented in Table 2.

"Object production accuracy" and "compressive strength" of the obtained three-dimensional object were evaluated in the same manners as in Example 5. The results are presented in Table 3.

**Table 2**

| | | Ex. 5 | | | Ex. 6 | | | Comp. Ex. 2 | | |
|---|---|---|---|---|---|---|---|---|---|---|
| | | Hydrogel precursor liquid | Chelate-containing liquid | Support forming liquid | Hydrogel precursor liquid | Chelate-containing liquid | Support forming liquid | Hydrogel precursor liquid | Chelate-containing liquid | Support forming liquid |
| Mineral | Synthetic hectorite | 0.8 | - | - | 0.8 | - | - | 0.8 | - | - |
| Polymerizable monomer | N,N-dimethylacrylamide | 4 | - | - | 4 | - | - | 4 | - | - |
| Surfactant | Sodium dodecyl sulfate | 0.01 | 0.01 | - | 0.01 | 0.01 | - | 0.01 | 0.01 | - |
| Polymerization initiator | 1-hydroxycyclohexylphenyl ketone | 0.015 | - | - | 0.015 | - | - | 0.015 | - | - |
| Chelate agent | 1-hydroxyethane-1,1-diphosphonic acid | - | 0.5 | - | - | 0.5 | - | - | - | - |
| Polymerization initiator | Tetramethylethylene diamine | 0.01 | - | - | 0.01 | - | - | 0.01 | | - |
| Pure water | | 20 | 20 | - | 20 | 20 | - | 20 | 20 | - |
| Curable material | Urethane acrylate | - | - | 10 | - | - | - | - | - | 10 |
| | Neopentylglycol hydroxypivalic acid ester di(meth)acrylate | - | - | 90 | - | - | - | - | - | 90 |
| Polymerization initiator | 1-hydroxycyclohexylphenyl ketone | - | - | 3 | - | - | - | - | - | 3 |
| Colorant | Blue pigment | - | - | 2 | - | - | - | - | - | 2 |
| Total (part by mass) | | 24.835 | 20.510 | 105.0 | 24.835 | 20.510 | - | 24.835 | 20.010 | 105.0 |
| Chelate agent content (% by mass) | | - | 2.44 | - | - | 2.44 | - | - | - | - |

**Table 3**

| | | Content of chelate agent in three-dimensional object (% by mass) | Evaluation result | |
|---|---|---|---|---|
| | | | Object production accuracy (%) | Compressive strength (MPa) at 70% compression |
| Ex. | 1 | 1.83 | 95 | 2.1 |
| | 2 | 1.83 | 94 | 3.0 |
| | 3 | 0.92 | 92 | 1.3 |
| | 4 | 0.37 | 93 | 0.9 |
| | 5 | 1.10 | 90 | 1.4 |
| | 6 | 1.10 | 70 | 1.2 |
| Comp. Ex. | 1 | 0 | 93 | 0.2 |
| | 2 | 0 | 75 | 0.3 |

From the results in Table 3, it can be understood that the three-dimensional objects of Examples 1 to 6 had an excellent object production accuracy and a high strength.

### Description of the Reference Numeral

10: three-dimensional object producing apparatus
11, 12: liquid material discharging head unit for an object
13: material discharging head unit for a support
14, 15: ultraviolet ray irradiator
16: object support substrate
18: support
20, 21, 22, 23: smoothing member
30: object
31, 32: support

## Claims

1. An organic-inorganic combined hydrogel precursor liquid comprising
water;
a polymerizable monomer;
a mineral; and
a chelate agent.

2. A three-dimensional object producing method comprising
producing a three-dimensional object using a first hydrogel precursor liquid and a second hydrogel precursor liquid,
wherein the first hydrogel precursor liquid and the second hydrogel precursor liquid contain the same components, but the blending ratio of the components in the first hydrogel precursor liquid and the blending ratio of the components in the second hydrogel precursor liquid are different from each other, and
wherein the first hydrogel precursor liquid and the second hydrogel precursor liquid comprise the components of the organic-inorganic combined hydrogel precursor liquid according to claim 1.

3. A material set for producing a three-dimensional object, the material set comprising:
an organic-inorganic combined hydrogel precursor liquid that comprises water, a polymerizable monomer and a mineral; and
a chelate agent-containing liquid.

4. A three-dimensional object producing method comprising:
applying the organic-inorganic combined hydrogel precursor liquid and a chelate agent-containing liquid according to claim 3 to the same position, to form a film,
wherein the three-dimensional object producing method repeats the applying a plurality of times.

5. The three-dimensional object producing method according to claim 4,
wherein a method for applying the organic-inorganic combined hydrogel precursor liquid and the chelate agent-containing liquid is at least any one of an inkjet method and a dispenser method, and
wherein the organic-inorganic combined hydrogel precursor liquid and the chelate agent-containing liquid are applied to a same position from different heads.

6. A three-dimensional object obtainable from a method according to any one of claims 2, 4 and 5 comprising an organic-inorganic combined hydrogel that comprises water as a main component, wherein the three-dimensional object comprises a chelate agent.

7. The three-dimensional object according to claim 6,
wherein a compressive strength of the three-dimensional object at 70% compression is 0.5 MPa or higher.

8. The three-dimensional object according to claim 6 or 7,
wherein a content of the chelate agent is 0.05% by mass or greater.

9. The three-dimensional object according to any one of claims 6 to 8, further comprising
a water-soluble polymer.

10. The three-dimensional object according to any one of claims 6 to 9,
wherein the three-dimensional object is an organ model.

## Patentansprüche

1. Organische-anorganische kombinierte Hydrogel-Vorläuferflüssigkeit, umfassend:
Wasser,
ein polymerisierbares Monomer;
ein Mineral; und
ein Chelat-Mittel.

2. Verfahren zum Herstellen eines dreidimensionalen Objekts, umfassend:
Herstellen eines dreidimensionalen Objekts unter Verwendung einer ersten Hydrogel-Vorläuferflüssigkeit und einer zweiten Hydrogel-Vorläuferflüssigkeit,
wobei die erste Hydrogel-Vorläuferflüssigkeit und die zweite Hydrogel-Vorläuferflüssigkeit dieselben Komponenten enthalten, wobei jedoch das Mischverhältnis der Komponenten in der ersten Hydrogel-Vorläuferflüssigkeit und das Mischverhältnis der Komponenten in der zweiten Hydrogel-Vorläuferflüssigkeit voneinander unterschiedlich sind, und
wobei die erste Hydrogel-Vorläuferflüssigkeit und die zweite Hydrogel-Vorläuferflüssigkeit die Komponenten der organischen-anorganischen kombinierten Hydrogel-Vorläuferflüssigkeit nach Anspruch 1 umfassen.

3. Materialsatz zum Herstellen eines dreidimensionalen Objekts, wobei der Materialsatz umfasst:
eine organische-anorganische kombinierte Hydrogel-Vorläuferflüssigkeit, welche Wasser, ein polymerisierbares Monomer und ein Mineral umfasst, und
eine Flüssigkeit, welche ein Chelat-Mittel enthält.

4. Verfahren zum Herstellen eines dreidimensionalen Objekts, umfassend:
Aufbringen der organischen-anorganischen kombinierten Hydrogel-Vorläuferflüssigkeit und einer ein Chelat-Mittel enthaltenden Flüssigkeit nach Anspruch 3 in derselben Position, um ein Film zu bilden,
wobei das Verfahren zum Herstellen eines dreidimensionalen Objekts das Aufbringen mehrmals wiederholt.

5. Verfahren zum Herstellen eines dreidimensionalen Objekts nach Anspruch 4,
wobei ein Verfahren zum Aufbringen der organischen-anorganischen kombinierten Hydrogel-Vorläuferflüssigkeit und der ein Chelat-Mittel enthaltenden Flüssigkeit mindestens eines von einem Tintenstrahlverfahren und einem Spenderverfahren ist, und
wobei die organische-anorganische kombinierte Hydrogel-Vorläuferflüssigkeit und die ein Chelat-Mittel enthaltende Flüssigkeit in derselben Position aus unterschiedlichen Köpfen aufgebracht werden.

6. Dreidimensionales Objekt, welches durch ein Verfahren nach einem der Ansprüche 2, 4 und 5 herstellbar ist, umfassend:
ein organisches-anorganisches kombiniertes Hydrogel, welches Wasser als Hauptkomponente umfasst,
wobei das dreidimensionale Objekt ein Chelat-Mittel umfasst.

7. Dreidimensionales Objekt nach Anspruch 6,
wobei eine Druckfestigkeit des dreidimensionalen Objekts bei 70 % Kompression 0,5 MPa oder mehr beträgt.

8. Dreidimensionales Objekt nach Anspruch 6 oder 7,
wobei ein Inhalt des Chelat-Mittels 0,05 Massen-% oder mehr beträgt.

9. Dreidimensionales Objekt nach einem der Ansprüche 6 bis 8, ferner umfassend ein wasserlösliches Polymer.

10. Dreidimensionales Objekt nach einem der Ansprüche 6 bis 9,
wobei das dreidimensionale Objekt ein Organmodell ist.

## Revendications

1. Liquide précurseur d'hydrogel combiné organique-inorganique comprenant :
de l'eau ;
un monomère polymérisable ;
un minéral ; et
un agent chélateur.

2. Procédé de fabrication d'un objet tridimensionnel comprenant :
la production d'un objet tridimensionnel en utilisant un premier liquide précurseur d'hydrogel et un deuxième liquide précurseur d'hydrogel,
dans lequel le premier liquide précurseur d'hydrogel et le deuxième liquide précurseur d'hydrogel contiennent les mêmes constituants, mais le rapport de mélange des constituants dans le premier liquide précurseur d'hydrogel et le rapport de mélange des constituants dans le deuxième liquide précurseur d'hydrogel sont différents l'un de l'autre, et
dans lequel le premier liquide précurseur d'hydrogel et le deuxième liquide précurseur d'hydrogel comprennent les constituants du liquide précurseur d'hydrogel combiné organique-inorganique selon la revendication 1.

3. Ensemble de matériaux pour fabriquer un objet tridimensionnel, l'ensemble de matériaux comprenant :
un liquide précurseur d'hydrogel combiné organique-inorganique qui comprend de l'eau, un monomère polymérisable, et un minéral ; et
un liquide contenant un agent chélateur.

4. Procédé de fabrication d'objet tridimensionnel comprenant :
l'application du liquide précurseur d'hydrogel combiné organique-inorganique et d'un liquide contenant un agent chélateur selon la revendication 3 à la même position, pour former un film,
dans lequel le procédé de fabrication de l'objet tridimensionnel répète l'application une pluralité de fois.

5. Procédé de fabrication d'objet tridimensionnel selon la revendication 4,
dans lequel un procédé d'application du liquide précurseur d'hydrogel combiné organique-inorganique et du liquide contenant un agent chélateur est au moins un parmi un procédé de jet d'encre et un procédé de distribution, et
dans lequel le liquide précurseur d'hydrogel combiné organique-inorganique et le liquide contenant un agent chélateur sont appliqués à une même position à partir de différentes têtes.

6. Objet tridimensionnel pouvant être obtenu par un procédé selon l'une quelconque des revendications 2, 4 et 5, comprenant
un liquide précurseur d'hydrogel combiné organique-inorganique qui comprend de l'eau en tant que constituant principal,
l'objet tridimensionnel comprenant un agent chélateur.

7. Objet tridimensionnel selon la revendication 6,
dans lequel une résistance à la compression de l'objet tridimensionnel à 70 % de compression est de 0,5 MPa ou plus.

8. Objet tridimensionnel selon la revendication 6 ou 7,
dans lequel une teneur de l'agent chélateur est de 0,05 % en masse ou plus.

9. Objet tridimensionnel selon l'une quelconque des revendications 6 à 8, comprenant en outre
un polymère soluble dans l'eau.

10. Objet tridimensionnel selon l'une quelconque des revendications 6 à 9,
dans lequel l'objet tridimensionnel est un modèle d'organe.
